# EUROPÄISCHE PATENTSCHRIFT

(11) **EP 1 099 452 B1**
(45) Veröffentlichungstag und Bekanntmachung des Hinweises auf die Patenterteilung: **23.05.2012**
(21) Anmeldenummer: 00122358.5
(22) Anmeldetag: 24.10.2000
(51) Int. Cl.: A61M 16/06

(54) **Atemmaske und Verfahren zur Herstellung derselben**
Respiratory mask and method for making the same
Masque respiratoire et méthode de fabrication

(30) Priorität: 12.11.1999 DE 19954517
(43) Veröffentlichungstag der Anmeldung: 16.05.2001
(60) Teilanmeldung: 10181552.0
(73) Patentinhaber: MAP Medizin-Technologie GmbH, 82152 Martinsried (DE)
(72) Erfinder: Lang, Bernd Christoph, 82166 Lochham (DE); Genger, Harald, 82319 Starnberg (DE)
(74) Vertreter: Heunemann, Dieter

(56) Entgegenhaltungen:
- EP-A- 0 602 424
- EP-A2- 1 147 782
- WO-A-98/48878
- WO-A-99/58198
- DE-A- 3 707 952
- DE-A- 19 735 359
- DE-A- 19 808 105
- GB-A- 790 677
- US-A- 4 454 881
- US-A- 5 676 133
- US-A- 5 884 624

## Beschreibung

Die Erfindung betrifft eine Atemmaske zur Zufuhr eines Atemgases zu einem Patienten sowie ein Verfahren zur Herstellung derselben.

Atemmasken der eingangs genannten Art finden insbesondere Anwendung im Bereich der Schlafmedizin. Über derartige Atemmasken kann dem Patienten ein Atemgas Umgebungsluft unter einem vorbestimmten Überdruck zugeführt werden. Hierdurch wird auf physiologisch vergleichsweise gut verträgliche Weise eine pneumatische Schienung der oberen Atemwege erreicht wodurch etwaigen Obstruktionen in diesem Bereich auf vorteilhafte Weise vorgebeugt werden kann.

Die im Bereich der Schlafmedizin angewandten Masken werden bei der Behandlung eines Patienten im Rahmen einer CPAP-Schlaftherapie täglich ca. 6 bis 8 Stunden getragen. Bei der Anwendung der bislang bekannten Masken besteht bei vielen Patienten das Problem, daß die gewünschte Dichtwirkung der Maske nur unter vergleichsweise großen Masken-Haltekräften erreicht werden kann und hierbei der Tragekomfort u.U. erheblich beeinträchtigt wird.

Die US 5 884 624 zeigt eine Atemmaske mit einer Dichtungseinrichtung, die aus einem Gelmaterial gefertigt und von einer Kunststofffolie überzogen ist.

Der Erfindung liegt die Aufgabe zugrunde, eine Atemmaske zur Zufuhr eines Atemgases unter Überdruck zu schaffen die sich durch einen hohen Tragekomfort auszeichnet und durch welche ein hoher Abdichtungsgrad auf zuverlässige Weise gewährleistet ist.

Diese Aufgabe wird erfindungsgemäß jeweils für sich durch eine Atemmaske mit wenigstens den in den unabhängigen Patentansprüchen und angegebenen Merkmalen gelöst.

Dadurch wird es auf vorteilhafte Weise möglich bei einer geringen Flächenpressung im Bereich der Gesichtskontaktzone durchgängig eine innige Anlage des inneren Dichtungsbereiches zu erreichen ohne daß die Gefahr besteht daß kleinere Positionsänderungen der Atemmaske gegenüber dem Gesicht des Patienten zu erheblichen Änderungen der Anpreßkräfte führen. In vorteilhafter Weise ist auch eine gegenüber herkömmlichen Atemmasken bessere Kompatibilität zu den individuellen Gesichtsformen der jeweiligen Patienten gegeben.

Eine sowohl im Hinblick auf einen hohen Tragekomfort sowie auch unter fertigungstechnischen Gesichtspunkten vorteilhafte Ausführungsform der Erfindung ist dadurch gegeben, daß der Maskenbasiskörper aus einem elastomeren Material gebildet ist. Im Hinblick auf eine hohe physiologische Verträglichkeit sowie gute Sterilisierbarkeit sind der Maskenbasiskörper und das Stirnauflageelement vorzugsweise aus einem Silikonmaterial gebildet. Auch die Dichtungseinrichtung ist vorzugsweise aus einem Silikonmaterial gebildet.

Eine besonders belastbare und im wesentlichen spaltfreie Koppelung von Maskenkörper und Dichtungseinrichtung ist dadurch gegeben, daß Maskenbasiskörper an die Dichtungseinrichtung angespritzt ist. Alternativ dazu ist es auch möglich, die Dichtungseinrichtung an den Maskenbasiskörper anzuspritzen sofern das zur Bildung des Maskenkörpers verwendete Material eine hinreichende Temperaturbeständigkeit aufweist.

Eine im Hinblick auf einen besonders hohen Tragekomfort sowie im Hinblick auf eine hohe Dichtwirkung vorteilhafte Ausführungsform der Erfindung ist dadurch gegeben, daß ein Versteifungselement vorgesehen ist, das dem Maskenbasiskörper und/oder der Dichtungseinrichtung eine vorbestimmte Gestalt verleiht. Dieses Versteifungselement ist vorzugsweise aus einem unter Wärmezufuhr verformbaren Material gebildet und behält nach Erkalten auf Raumtemperatur die ihm verliehene Gestalt unter allenfalls elastischer Verformung weitgehend bei.

Eine besonders individuelle Anpassung der Atemmaske an die Kopfform des Patienten wird gemäß einer besonders bevorzugten Ausführungsform der Erfindung dadurch erreicht, daß sich das Versteifungselement bis in ein Stirnauflageelement hinein erstreckt.

In dem Maskenbasiskörper und/oder dem Stirnauflageelement ist vorzugsweise ein Aufnahmeabschnitt ausgebildet, zur Aufnahme des Versteifungselementes. Dieser Aufnahmeabschnitt kann durch eine komplementär zum Querschnitt des Versteifungselementes ausgebildete Ausnehmung gebildet sein. Vorzugsweise ist das Versteifungselement eng sitzend in dieser Ausnehmung aufgenommen, bedarfsweise eingeklebt.

An dem Stirnauflageelement ist vorzugsweise eine Koppelungseinrichtung vorgesehen, zum Anschluß eines Maskenhaltebandes.

Die Dichtungseinrichtung zur Abdichtung des Maskenbasiskörpers kann ein aus einem gelartig ausgehärteten Elastomermaterial gebildetes Organ aufweisen, wobei der Elastomermaterial-Abschnitt vorzugsweise mit einer Hautschicht überzogen ist die aus einem elastomeren Material gebildet ist. Hierdurch wird auf überraschend wirkungsvolle Weise einer ungünstigen Bildung von Falten vorgebeugt. Die Hautschicht ist vorzugsweise aus einem höher vernetzten Silikonkautschukmaterial gebildet.

Die genannte Hautschicht kann auf fertigungstechnisch vorteilhafte Weise durch Auftrag einer dünnen Silikonmaterialschicht auf einen entsprechenden Formraumabschnitt eines Formwerkzeuges gebildet werden, wobei später das gelartig aushärtende Silikonmaterial in den entsprechend vorbereiteten, verbleibenden Formraumabschnitt eingespritzt ist und hierbei eine innige Verbindung mit dem Hautabschnitt erreicht wird.

Die insbesondere unter fertigungstechnischen Gesichtspunkten vorteilhafte Ausführungsform der Atemmaske mit einem Maskenbasiskörper der einen Maskeninnenraum begrenzt, einem Atemgaszuleitungsabschnitt zur Zuleitung von Atemgas zu dem Maskeninnenraum, und einer nachgiebigen Dichtungseinrichtung zur Abdichtung des Maskenbasiskörpers ist dadurch gegeben, daß der Maskenbasiskörper an die Dichtungseinrichtung angespritzt ist.

Ein im Hinblick auf einen hohen Tragekomfort besonders vorteilhaftes Polsterelement wird insbesondere dadurch erreicht, daß die Dichtungseinrichtung ein Dichtlippenelement aufweist das aus einem Silikonmaterial mit hohem Vernetzungsgrad gefertigt ist, und daß an dieses Dichtlippenelement ein Polsterkörper angekoppelt ist, der aus einem Silikonmaterial niedrigen Vernetzungsgrades gefertigt ist. Vorzugsweise ist der Polsterkörper zumindest im Bereich der Gesichtsauflagefläche mit einer elastomeren Haut überzogen.

Vorzugsweise ist ein einwärts gerichtet umlaufendes Dichtlippenelement einstückig mit dem Polsterkörper ausgebildet.

Unter fertigungstechnischen Gesichtspunkten wird die eingangs angegebenen Aufgabe auch durch ein Verfahren zur Herstellung einer Atemmaske gelöst, bei welchem im Rahmen eines ersten Silikoneinspritzvorganges Silikonmaterial in einen Formraumabschnitt eingespritzt wird der komplementär zu einer Masken-Dichtungseinrichtung ausgebildet ist, wobei im Rahmen eines nachfolgenden Verfahrensschrittes ein Maskenkörperseitiger Verbindungsbereich zunächst entformt und anschließend in einen komplementär zu einem Maskenkörper ausgebildeten Formraumabschnitt eingebracht wird, und erst anschließend ein Kunststoffmaterial in den, zum Maskenkörper komplementären Formraum eingespritzt wird.

Weitere Einzelheiten und Merkmale der Erfindung ergeben sich aus der nachfolgenden Beschreibung mehrerer bevorzugter Ausführungsbeispiele in Verbindung mit der Zeichnung. Es zeigen:
- **Fig. 1a:**: eine vereinfachte perspektivische Ansicht einer Atemmaske mit integriertem Stirnauflageelement;
- **Fig. 1b:**: eine vereinfachte Detailansicht zur Erläuterung eines Befestigungselementes zur Koppelung einer Kopfbandanordnung mit der Atemmaske;
- **Fig. 1c:**: eine vereinfachte perspektivische Ansicht zur Erläuterung des Aufbaus eines Stirnauflageelementes, das integral mit dem Maskenbasiskörper aus einem elastomeren Material gebildet ist;
- **Fig. 1d:**: eine Skizze zur Erläuterung der Gestalt der Maskenpolsterung für den Be- reich des Nasenrückens eines Patienten;
- **Fig. 2:**: eine perspektivische Ansicht eines Versteifungselementes für eine Atemmas- ke, das sich bis in den Stirnauflagebereich hinein erstreckt;
- **Fig. 3a:**: eine weitere Ausführungsform eines Versteifungselementes für einen Mas- kenbasiskörper, das hier aus einem Drahtmaterial mit im wesentlichen kreis- zylindrischem Querschnitt gefertigt ist;
- **Fig. 3b:**: eine perspektivische Detailansicht, durch den Dichtlippenbereich eines Mas- kenelementes im Bereich des Nasenrückens mit daran angebrachtem Ver- steifungselement im wesentlichen gem. Fig. 3a;
- **Fig. 4:**: eine vereinfachte Schnittansicht durch den Dichtlippenbereich einer Atem- maske mit einem Gel-Polsterkörper sowie einem unmittelbar benachbart an- geordneten Versteifungselement;
- **Fig. 5:**: eine vereinfachte Schnittansicht durch einen Dichtlippenbereich mit ange- spritztem Maskenbasiskörper;
- **Fig. 6:**: eine vereinfachte Schnittansicht durch eine erfindungsgemäße Dichtlippenstruktur mit einem an eine Hauptdichtlippe angespritzten Gelkörper dessen dem Patienten zugewandte Außen- seite mit einer elastomeren Hautschicht überzogen ist:

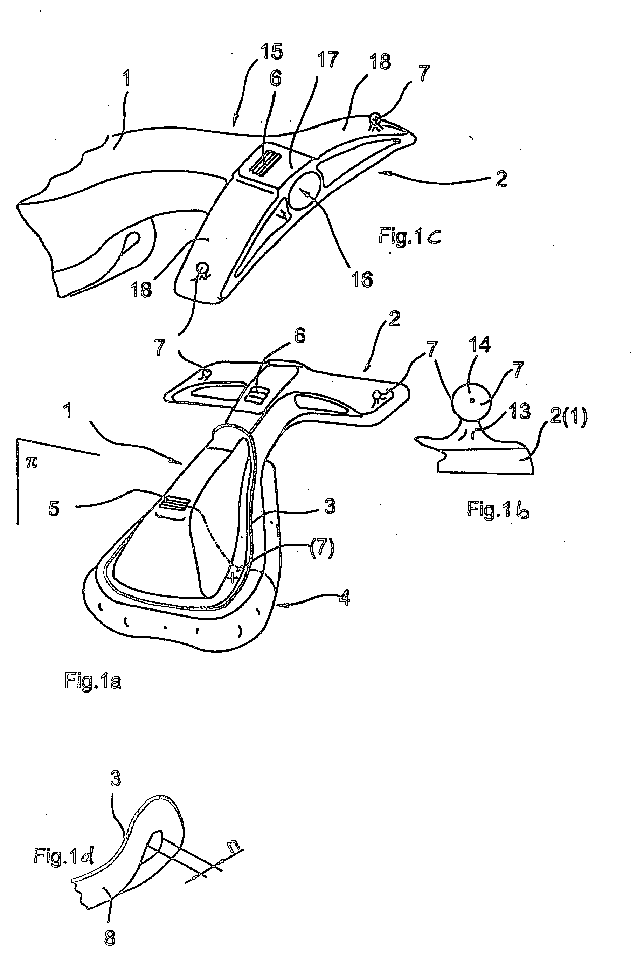

Fig. 1 zeigt eine erste bevorzugte Ausführungsform einer Atemmaske, die einen Maskenbasiskörper 1 mit einem integral damit ausgebildeten Stirnauflageelement 2 aufweist. Bei der dargestellten Ausführungsform sind der Maskenbasiskörper 1 und das Stirnauflageelement 2 aus einem elastomeren Material, insbesondere Silikonkautschuk gebildet. Der Maskenbasiskörper 1 ist durch ein umlaufendes Versteifungselement 3 verstärkt. Bei der gezeigten Ausführungsform besteht das Versteifungselement 3 aus einem Kunststoffmaterial, das hinsichtlich seiner räumlichen Gestalt im erwärmten Zustand individuell auf den Patienten abgestimmt wurde. Das Versteifungselement 3 ist in einer hier nur andeutungsweise dargestellten umlaufenden Nut an dem Maskenbasiskörper 1 fixiert.

Der Maskenbasiskörper 1 weist weiterhin ein integral mit diesem ausgebildetes Dichtlippenelement 4 auf. Das Dichtlippenelement 4 und der Maskenbasiskörper 1 sind ebenfalls integral ausgebildet, so daß zwischen dem Dichtlippenelement 4 und dem Maskenbasiskörper keine Spaltbereiche gebildet sind.

In dem Maskenbasiskörper 1 sind weiterhin Auslaßöffnungen 5, 6 gebildet, über welche permanent unter Überdruck stehendes Gas aus dem Innenbereich der Maske nach außen hin entweichen kann. Die Auslaßöffnungen 5, 6 sind hier durch kleine, in den Maskenbasiskörper 1 eingeformte Schlitze gebildet. Bei der hier dargestellten Ausführungsform sind die Schlitze durch dünne, ebenfalls integral mit dem Maskenbasiskörper 1 gebildete Stege voneinander getrennt. Alternativ hierzu ist es auch möglich, eine Blendenstruktur, die einen definierten Durchgangsquerschnitt aufweist, in eine entsprechende in dem Maskenbasiskörper 1 gebildete Aufnahmeöffnung einzusetzen, insbesondere lösbar einzuklipsen. Die Anordnung der Auslaßöffnungen 5, 6 ist bei der gezeigten Atemmaske derart getroffen, daß im Inneren der Atemmaske keine deutlich wahrnehmbaren Luftströmungen seitens des Patienten auftreten. Der Durchgangsquerschnitt, der dem Stirnauflageelement 2 benachbarten Auslaßöffnungen 6 ist bei der gezeigten Ausführungsform größer bemessen, als der Durchgangsquerschnitt der im Nasenspitzenbereich vorgesehenen Auslaßöffnungen 5. Das Größenverhältnis ist derart festgelegt, daß ca. 2/3 der ausströmenden Luft durch die dem Stirnbereich benachbarten Auslaßöffnungen 6 und das verbleibende Drittel durch die vorderen Auslaßöffnungen 5 abströmt. Hierdurch wird ein optimaler Austausch der verbrauchten Atemluft bei geringen Zuglufterscheinungen erreicht.

Das Stirnauflageelement 2 ist derart ausgebildet, daß dieses großflächig und unter gleichmäßiger Flächenpressung auf dem Stirnbereich des Patienten aufliegt. Das Stirnauflageelement 2 ist mit Halteorganen 7 versehen, an welchen ein entsprechender Koppelungsabschnitt einer Maskenhalterung, insbesondere einer Kopfbandanordnung oder Halte-Mütze anbringbar ist. Eine bevorzugte Ausführungsform dieser Halteorgane 7 ist in Fig. 1b dargestellt. Der Maskenbasiskörper 1 ist derart ausgebildet, daß der Übergangsbereich zwischen dem Maskenbasiskörper und dem Dichtlippenelement einen im wesentlichen der Gesichtsform des Patienten folgenden Höhenverlauf aufweist. Insbesondere ist der Maskenbasiskörper im Bereich des Nasenrückens des Patienten derart eingezogen ausgebildet, daß das Dichtlippenelement - wie in Fig. 1d angedeutet - sattelartig über den Nasenrücken des Patienten geführt werden kann.

Eine vorbestimmte Anpreßkraft wird vorzugsweise über eine Kopfbandanordnung erzeugt, die wenigstens ein um den Hinterkopfbereich eines Patienten herumgeführtes Gurtband aufweist. Dieses Gurtband kann über entsprechende Endstücke, bzw. Koppelungsorgane auf einfache Weise mit der Atemmaske über die bereits genannten Halteorgane 7 gekoppelt werden. Diese Halteorgane 7 können - wie in Fig. 1b dargestellt - integral mit dem Maskenbasiskörper 1, bzw. mit dessen Stirnauflageelement 2, ausgebildet sein. Im vorliegenden Falle weisen die Halteorgane einen Schaftabschnitt 13 und einen daran angeformten Kugelkopfabschnitt 14 auf. Der Durchmesser des Kopfabschnittes 14 ist größer als der Durchmesser des Schaftabschnittes 13. Derartige Halteorgane sind vorzugsweise an mehreren Stellen der Atemmaske 1, insbesondere auch im vorderen Bereich des Maskenbasiskörpers 1 - wie angedeutet - angeordnet.

Wie aus Fig. 1c hervorgeht, ist bei der dargestellten Atemmaske das Stirnauflageelement 2 derart langgestreckt ausgebildet, daß sich eine gleichmäßige Verteilung der Auflagekräfte ergibt. Das Stirnauflageelement 2 ist über einen Halsabschnitt mit dem Maskenbasiskörper 1 gekoppelt. Der Maskenbasiskörper 1, der Halsabschnitt 15 und das Stirnauflageelement 2 sind hier einstückig ausgebildet. Durch den Halsabschnitt 15 hindurch erstreckt sich ein Atemgaskanal 16, der bis in einen - ebenfalls mit dem Stirnauflageelement 2 ausgebildeten Anschlußabschnitt 17 mündet. Der Innendurchmesser des Anschlußabschnittes 17 ist derart ausgebildet, daß eine entsprechende Anschlußstruktur eines Atemgasschlauches unmittelbar in den Anschlußabschnitt 17 eingesteckt werden kann. Im Bereich des Anschlußabschnittes 17 sind auch die vorangehend bereits unter Bezugnahme auf Fig. 1a erläuterten Auslaßöffnungen 6 vorgesehen, über welche ein vorbestimmter Gasstrom nach außen entweichen kann. Das Stirnauflageelement 2 weist bei der dargestellten Ausführungsform zwei Flügelabschnitte 18 auf, durch welche das Stirnauflageelement 2 noch weiter versteift wird, und zudem die über die Halteorgane 7 eingeleiteten Zugkräfte gleichmäßig übertragen werden. Auch die Flügelabschnitte 18 sind bei der dargestellten Ausführungsform einstückig mit dem Maskenbasiskörper, dem Halsabschnitt 15 und dem Stirnauflageelement 2 aus einem Elastomermaterial gebildet. Durch entsprechende Querschnittsgestaltung der Flügelabschnitte kann das Verformungsverhalten des Stimauflageelementes gezielt beeinflußt werden.

In Fig. 1d ist lediglich andeutungsweise dargestellt, wie die Dichtungseinsrichtung 4 sattelartig dem Gesichtsprofil des Patienten folgend über den Nasenrücken herumgeführt ist. Der Anpreßdruck der Dichtungseinsrichtung im Bereich des Nasenrückens kann auf vorteilhafte Weise durch das Versteifungselement 3 beeinflußt werden, indem die hier andeutungsweise skizzierte Nasenrückenweite n durch das Versteifungselement 3 definiert festgelegt wird. Hierdurch wird vermieden, daß der Nasenrückenbereich des Patienten über die Dichtungseinsrichtung in unangenehmer Weise zu stark zusammengedrückt wird. Durch die Kombination von Dichtungseinsrichtung 4 und Versteifungsetement 3 wird es auf vorteilhafte Weise möglich, einen besonders hohen Tragekomfort sowie eine hohe Dichtigkeit der Maske zu erreichen, da die Dichtungseinsrichtung 4 vor Auflage auf der Gesichtsfläche des Patienten bereits in eine weitgehend der Gesichtskontur des Patienten entsprechende Gestalt aufweist. Die Kombination von Versteifungskörper 3 und Dichtungseinsrichtung kann auch unabhängig von der integralen Ausbildung von Maskenbasiskörper 1 und Stirnauflageelement 2 Anwendung finden.

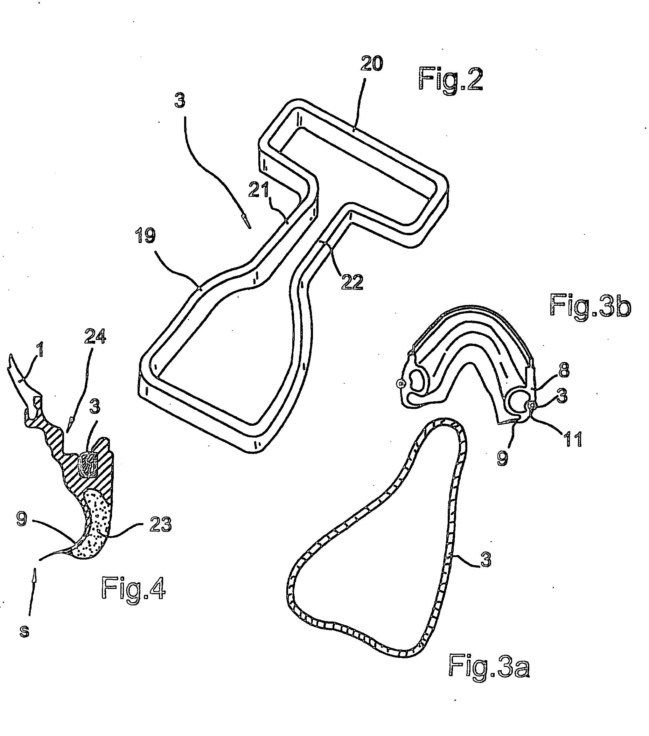

In Fig. 2 ist eine bevorzugte Ausführungsform des Versteifungselementes 3 dargestellt, das hier sowohl einen der Gesichtskontur des Patienten im Bereich der Nase angepaßten, bzw. anpaßbaren Abschnitt 19 sowie einen an die Stirnkontur des Patienten angepaßten Abschnitt 20 aufweist.

Die beiden Abschnitte 19, 20 sind über Schenkel 21, 22 miteinander gekoppelt. Das Versteifungselement 3 ist hier aus einem thermoverformbaren Kunststoffmaterial gebildet, das gemeinsam mit einem entsprechenden Maskenbasiskörper mit integralem Stirnauflageelement an den Patienten angepaßt wird. Nach Erkalten des entsprechenden Kunststoffmaterials behält das Versteifungselement 3 die ihm individuell gegebene Form dauerhaft bei. Das Versteifungselement 3 kann bedarfsweise von dem Maskenbasiskörper 1 und dem Stirnauflageelement 2 (vgl. hierzu Fig. 1a) entfernt werden und in einen neuen entsprechenden Maskenbasiskörper eingesetzt werden, so daß dieser im wesentlichen die gleiche Gestalt wie der ursprüngliche Maskenkörper erhält. Durch die hier dargestellte Ausführungsform des Versteifungselementes 3 wird es möglich, insbesondere das Höhenniveau des Stirnauflageelementes und des Maskenbasiskörpers 1 exakt aufeinander abzustimmen, so daß die entsprechend individuell vorgeformte Atemmaske bereits vor Aufsetzen auf das Gesicht des Patienten eine weitgehend dessen Gesichtskontur entsprechende Gestalt aufweist.

Alternativ zu der in Fig. 2 beschriebenen Ausführungsform des rahmenartig ausgebildeten Versteifungselementes 3 ist es auch möglich, dieses beispielsweise aus einem Drahtmaterial zu fertigen, wie in Fig. 3a vereinfacht angedeutet. Hierbei wird vorzugsweise ein nicht korrodierender Stahlwerkstoff verwendet, der über entsprechende Biegewerkzeuge in die gewünschte Form gebogen wird. Dieses durch ein Drahtbügel gebildete Versteifungselement 3 kann dann, wie in Fig. 3b angedeutet, mit dem Maskenbasiskörper 1 bzw. mit dessen Dichtungsstruktur gekoppelt werden. Vorzugsweise weist der Maskenbasiskörper 1, bzw. die Dichtungsstruktur 8, 9 entsprechende Ausnehmungen 11 auf, in die das Versteifungselement 3 eingefügt werden kann. Bei der hier in Fig. 3b andeutungsweise dargestellten Dichtungsstruktur ist lediglich der über den Nasenrücken des Patienten geführte Dichtungsstrukturbereich dargestellt.

In Fig. 4 ist eine Dichtungsstruktur für eine Atemmaske dargestellt, die hier eine Gesichtsdichtlippe 9 aufweist, die zu einer äußerst dünnen Dichtlippenspitze s ausläuft. Die Wandungsdicke der Gesichtsdichtlippe 9 nimmt zu einem Wurzelbereich der Gesichtsdichtlippe hin allmählich zu. An den Außenbereich der Gesichtsdichtlippe 9 ist ein Polsterkörper 23 angeformt, der hier aus einem gelartig ausgehärteten Material - hier Silikonkautschuk mit niedrigem Vernetzungsgrad - gebildet ist. Dieser Polsterkörper 23 stützt den Maskenbasiskörper 1 gemeinsam mit der Dichtlippe 9 auf dem Gesicht des Patienten ab. Hier dargestellt ist der Maskenbasiskörper 1 separat von der Dichtungsstruktur 24 ausgebildet. Die Dichtungsstruktur ist ähnlich wie bei den vorangehend beschriebenen Ausführungsformen über ein Versteifungselement 3 bereits in eine der jeweiligen Gesichtskontur des Patienten entsprechende räumliche Gestalt vorgeformt. Der Außenbereich des Polsterkörpers 23 ist mit einer Hautschicht überzogen, die ein Ankleben des Polsterkörpers 23 an der Gesichtsoberfläche des Patienten verhindert. Die Oberfläche dieser Hautschicht weist hier eine vorbestimmte samtartige Mikrorauhigkeit auf.

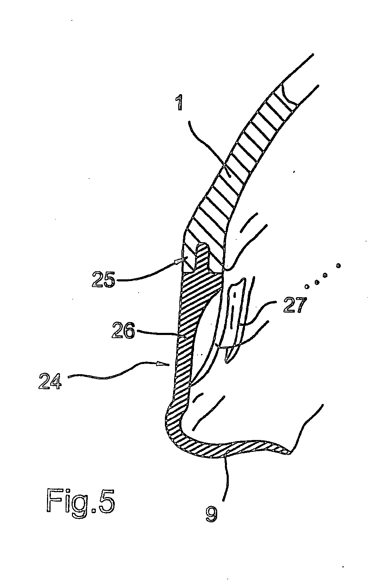

In Fig. 5 ist eine Dichtungsstruktur 24 für eine Atemmaske dargestellt, die hier ebenfalls eine vom Außenbereich der Atemmaske sich im wesentlichen radial einwärts erstreckende dünn auslaufende Gesichtsdichtlippe 9 aufweist. Der Krümmungsverlauf der Gesichtsdichtlippe 9 in der gezeigten Schnittebene (Ebene π in Fig. 1a) ist derart gewählt, daß sich die Dichtlippe vergleichsweise gut an das Profil des Patienten anpassen kann. Die Dichtungsstruktur 24 und der Maskenbasiskörper 1 sind hier aus unterschiedlichen Werkstoffen gebildet. So ist bei dem gezeigten Ausführungsbeispiel die Dichtungsstruktur aus einem Silikonmaterial und der Maskenbasiskörper aus einem thermoplastischen Kunststoffmaterial gebildet. Der Maskenbasiskörper 1 ist in einem speziellen Formwerkzeug unmittelbar an die im Rahmen eines vorangegangenen Silikonspritzschrittes gebildete Dichtlippenstruktur angespritzt, wodurch eine im wesentlichen unlösbare und spaltfreie Verbindung zwischen der Dichtungsstruktur 24 und dem Maskenbasiskörper erreicht wird. Im Bereich der Übergangsstelle zwischen der Dichtungsstruktur 24 und dem Maskenbasiskörper ein umlaufender Stegbereich 25 vorgesehen, über welchen die Festigkeit der Verbindung zwischen Maskenbasiskörper und Dichtungsstruktur 24 noch weiter verbessert wird.

Das Verformungsverhalten der Dichtungsstruktur 24 wird durch eine definierte Gestaltung der Wanddicken der Gesichtsdichtlippe 9 sowie des sich daran anschließenden, zum Maskenbasiskörper 1 fortsetzenden Wandungsabschnitt 26 definiert beeinflußt. Weiterhin sind zahlreiche integral mit der Dichtungsstruktur 24 ausgebildete Stege 27 vorgesehen, deren Länge und Wandstärke ebenfalls das Verformungsverhalten der Dichtungsstruktur 24 definiert beeinflußt. Obgleich bei der hier dargestellten struktur kein Versteifungselement vorgesehen ist, ist es auch möglich, mittels des Versteifungselementes eine noch individuellere Anpassung an die Gesichtskontur des Patienten zu erreichen.

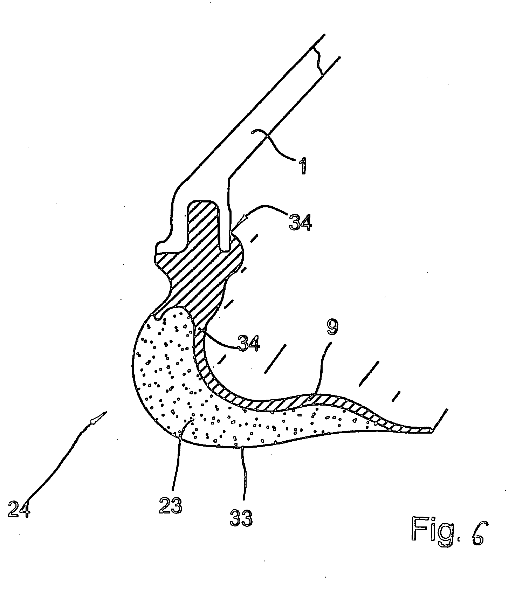

In Fig. 6 ist eine weitere bevorzugte Ausführungsform einer Dichtungsstruktur für eine Atemmaske gezeigt, die ähnlich, wie die bereits in Verbindung mit Fig. 4 beschriebene Dichtungsstruktur eine zur Gesichtsfläche des Patienten hin dünn auslaufende Gesichtsdichtlippe 9 aufweist. Diese Gesichtsdichtlippe 9 weist eine vergleichsweise hohe Tragfähigkeit auf. Die der Gesichtsfläche des Patienten zugewandte Außenseite der Gesichtsdichtlippe 9 ist zum überwiegenden Teil mit einem Polsterkörper 23 überzogen, der aus einem Elastomermaterial mit niedrigem Vernetzungsgrad gebildet ist (Gelmaterial). Die Außenseite dieses Polsterkörpers 23 ist mit einer samtartig rauhen dünnen Hautschicht 33 überzogen. Diese dünne Hautschicht 33 ist gem. einer besonders bevorzugten Ausführungsform der Erfindung aus einem Elastomermaterial, insbesondere ebenfalls Silikonmaterial mit höherem Vernetzungsgrad gebildet. Hierdurch wird es möglich, den Gelkörper zu verformen, ohne, daß an dessen Außenseite Kräuselfalten auftreten. Der Querschnitt der Dichtungsstruktur 24 ist derart gewählt, daß ein Teil der über die dünne Hautschicht 33 in dem Polsterkörper 23 eingeleiteten Aufstandskräfte sich unmittelbar in einen Wurzelbereich 24 der Gesichtsdichtlippe 9 von hier aus in den Maskenbasiskörper 1 überträgt. Der Maskenbasiskörper ist bei der hier dargestellten Ausführungsform mit der Dichtungsstruktur 24 über eine Nut/Federstruktur verklebt. Eine innere Fügungsstelle 34 zwischen dem Maskenbasiskörper 1 und der Dichtungsstruktur 24 ist durch eine an die Dichtungsstruktur 24 angeformte Nase abgedichtet.

## Patentansprüche

1. Atemmaske mit:
- einem Maskenbasiskörper (1) der einen Maskeninnenraum begrenzt,
- einem Atemgaszuleitungsabschnitt zur Zuleitung von Atemgas zu dem Maskeninnenraum,
und
- einer Dichtungseinrichtung (4, 24) zur Abdichtung des Maskenbasiskörpers, wobei
die Dichtungseinrichtung (4, 24) ein Dichtlippenelement (9) aufweist, das mit hohem Vernetzungsgrad gefertigt ist, und dass an dieses Dichtlippenelement ein Polsterkörper (8, 23) angekoppelt ist, der mit einem niedrigen Vernetzungsgrad gefertigt ist,
**dadurch gekennzeichnet,**
**dass** die Dichtungseinrichtung (4, 24) an den Maskenbasiskörper (1), oder der Maskenbasiskörper (1) an die Dichtungseinrichtung (4, 24) angespritzt ist, und dass das Dichtlippenelement und der Polsterkörper aus einem Silikonmaterial gefertigt sind.

2. Atemmaske nach Anspruch 1, **dadurch gekennzeichnet, dass** der Polsterkörper zumindest im Bereich der Gesichtsauflagefläche mit einer elastomeren Haut überzogen ist.

3. Atemmaske nach Anspruch 1, **dadurch gekennzeichnet, dass** der Maskenbasiskörper aus einem elastomeren Material gebildet ist.

4. Atemmaske nach wenigstens einem der Ansprüche 1 bis 3, **dadurch gekennzeichnet, dass** ein Versteifungselement vorgesehen ist, das dem Maskenbasiskörper und/oder der Dichtungseinrichtung eine vorbestimmte Gestalt verleiht.

5. Atemmaske nach Anspruch 4, **dadurch gekennzeichnet, dass** das Versteifungselement aus einem unter Wärmezufuhr verformbaren Material gebildet ist und bei Erkalten auf Raumtemperatur die ihm verliehene Gestalt weitgehend beibehält.

6. Atemmaske nach wenigstens einem der Ansprüche 4 oder 5, **dadurch gekennzeichnet, dass** in dem Maskenbasiskörper und/oder dem Stirnauflageelement ein Aufnahmeabschnitt ausgebildet ist, zur Aufnahme des Versteifungselementes.

7. Atemmaske nach Anspruch 2, **dadurch gekennzeichnet, dass** die Hautschicht aus einem höher vernetzten Silikonkautschukmaterial, insbesondere durch einen Tauchvorgang gebildet ist.

8. Atemmaske nach Anspruch 2, **dadurch gekennzeichnet, dass** die Hautschicht durch Auftrag einer dünnen Silikonmaterialschicht auf einen entsprechenden Formraumabschnitt eines Formwerkzeuges gebildet ist, und dass das gelartig aushärtende Silikonmaterial in den entsprechend vorbereiteten Formraumabschnitt eingespritzt ist.

9. Verfahren zur Herstellung einer Atemmaske mit einem Maskenbasiskörper (1) der einen Maskeninnenraum begrenzt, einem Atemgaszuleitungsabschnitt zur Zuleitung von Atemgas zu dem Maskeninnenraum und einer Dichtungseinrichtung (4, 24) zur Abdichtung des Maskenbasiskörpers (1) mit den Schritten
- Herstellen eines Dichtlippenelements (9) aus einem Silikonmaterial mit hohem Vernetzungsgrad,
- Herstellen der Dichtungseinrichtung (4, 24) durch Ankoppeln eines Polsterkörpers (8, 23) aus einem Silikonmaterial niedrigen Vernetzungsgrades an dieses Dichtlippenelement (9),
wobei die Dichtungseinrichtung (4, 24) an den Maskenbasiskörper (1), oder der Maskenbasiskörper (1) an die Dichtungseinrichtung (4, 24) angespritzt ist.

## Claims

1. A breathing mask comprising:
- a mask base body (1) limiting the interior of a mask,
- a respiratory gas supply portion for supplying respiratory gas to the interior of the mask, and
- a sealing means (4, 24) for sealing the mask base body,
wherein
the sealing means (4, 24) comprises a sealing lip element (9) having a high cross-linking capability and that a cushioning body (8, 23) having a low cross-linking capability is coupled to said sealing lip element,
**characterized in that** the sealing means (4, 24) is injection molded to the mask base body (1) or the mask base body (1) is injection molded to the sealing means (4, 24) and that the sealing lip element and the cushioning body are made from a silicone material.

2. The breathing mask according to claim 1, **characterized in that** at least the face rest area of the cushioning body is covered with an elastomeric skin.

3. The breathing mask according to claim 1, **characterized in that** the mask base body is formed from an elastomeric material.

4. The breathing mask according to at least one of claims 1 to 3, **characterized in that** a stiffening element is provided which gives the mask base body and/or the sealing means a predetermined shape.

5. The breathing mask according to claim 4, **characterized in that** the stiffening element is formed from a material which is deformable when heat is applied and which maintains the given shape to a large extent when cooling down to room temperature.

6. The breathing mask according to at least one of claims 4 or 5, **characterized in that** a receiving portion for receiving the stiffening element is formed in the mask base body and/or the forehead rest element.

7. The breathing mask according to claim 2, **characterized in that** the skin layer is made from a relatively highly cross-linked silicone rubber material in particular by an immersion process.

8. The breathing mask according to claim 2, **characterized in that** the skin layer is formed by applying a thin silicone material layer onto a respective mold portion of a molding tool and that the silicone material which cures so as to form a gel is injected into the respectively prepared mold portion.

9. A method for manufacturing a breathing mask comprising a mask base body (1) limiting the interior of a mask, a respiratory gas supply portion for supplying respiratory gas to the interior of the mask, and a sealing means (4, 24) for sealing the mask base body (1), the method comprising the steps:
- manufacturing the sealing lip element (9) from a silicone material having a high cross-linking capability,
- manufacturing the sealing lip means (4, 24) by coupling a cushioning body (8, 23) made from a silicone material having a low cross-linking capability to said sealing lip element (9),
wherein the sealing means (4, 24) is injection molded to the mask base body (1) or the mask base body (1) is injection molded to the sealing means (4, 24).

## Revendications

1. Masque respiratoire comprenant :
- un corps de base (1) délimitant un espace intérieur,
- une zone d'amenée de gaz de respiration, dévolue à la délivrance de gaz de respiration audit espace intérieur du masque, et
- un dispositif d'étanchement (4, 24) conçu pour assurer l'étanchéité dudit corps de base dudit masque, sachant que
ledit dispositif d'étanchement (4, 24) présente un élément à lèvre d'étanchement (9) fabriqué avec haut degré de réticulation, un corps de rembourrage (8, 23), fabriqué avec faible degré de réticulation, étant implanté sur cet élément à lèvre d'étanchement, **caractérisé par le fait**
**que** le dispositif d'étanchement (4, 24) est rapporté par injection sur le corps de base (1) du masque, ou bien ledit corps de base (1) du masque est rapporté par injection sur ledit dispositif d'étanchement (4, 24) ; et
**que** l'élément à lèvre d'étanchement et le corps de rembourrage sont fabriqués en un matériau silicone.

2. Masque respiratoire selon la revendication 1, **caractérisé par le fait que** le corps de rembourrage est revêtu d'une peau en élastomère, au moins dans la région de la surface en applique contre le visage.

3. Masque respiratoire selon la revendication 1, **caractérisé par le fait que** le corps de base dudit masque est fabriqué en un matériau élastomère.

4. Masque respiratoire selon au moins l'une des revendications 1 à 3, **caractérisé par** la présence d'un élément de rigidification donnant une configuration prédéterminée au corps de base dudit masque et/ou au dispositif d'étanchement.

5. Masque respiratoire selon la revendication 4, **caractérisé par le fait que** l'élément de rigidification est constitué d'un matériau déformable par apport de chaleur et conserve amplement, lors d'un refroidissement jusqu'à température ambiante, la configuration qui lui a été donnée.

6. Masque respiratoire selon au moins l'une des revendications 4 ou 5, **caractérisé par le fait qu'**une zone réceptrice, conçue pour recevoir l'élément de rigidification, est ménagée dans le corps de base dudit masque et/ou dans l'élément en applique contre le front.

7. Masque respiratoire selon la revendication 2, **caractérisé par le fait que** la couche de peau est constituée d'un matériau en caoutchouc au silicone à réticulation plus intense, notamment par une opération d'immersion.

8. Masque respiratoire selon la revendication 2, **caractérisé par le fait que** la couche de peau est formée par dépôt d'une mince couche de matériau silicone sur une région correspondante, constituant la cavité de moulage d'un outil de moulage ; et **par le fait que** ledit matériau silicone, durcissant à la manière d'un gel, est injecté dans ladite région formant cavité de moulage et apprêtée en conséquence.

9. Procédé de fabrication d'un masque respiratoire comprenant un corps de base (1) délimitant un espace intérieur, une zone d'amenée de gaz de respiration, dévolue à la délivrance de gaz de respiration audit espace intérieur du masque, et un dispositif d'étanchement (4, 24) conçu pour assurer l'étanchéité dudit corps de base (1) dudit masque, englobant les étapes consistant à
- fabriquer un élément à lèvre d'étanchement (9) en un matériau silicone à haut degré de réticulation,
- produire le dispositif d'étanchement (4, 24) en implantant, sur cet élément à lèvre d'étanchement (9), un corps de rembourrage (8, 23) en un matériau silicone à faible degré de réticulation,
ledit dispositif d'étanchement (4, 24) étant rapporté par injection sur le corps de base (1) du masque, ou bien ledit corps de base (1) dudit masque étant rapporté par injection sur ledit dispositif d'étanchement (4, 24).
